# FASCICULE DE BREVET EUROPEEN

(11) **EP 1 738 799 B1**
(45) Date de publication et mention de la délivrance du brevet: **20.08.2008**
(21) Numéro de dépôt: 05292243.2
(22) Date de dépôt: 24.10.2005
(51) Int. Cl.: A61Q 5/06, A61Q 5/10, A61K 8/49

(54) **Utilisation de composés azoïques polycationiques en teinture des fibres kératiniques**
Verwendung von polykationischen Azoverbindungen zum Färben keratinischer Fasern
Use of polycationic azo compounds for dyeing of keratinic fibres

(30) Priorité: 31.05.2005 US 139626
(43) Date de publication de la demande: 03.01.2007
(73) Titulaire: L'ORÉAL, 75008 Paris (FR)
(72) Inventeur: Lagrange, Alain, 77700 Coupvray (FR); David, Hervé, 94210 La Varenne St-Hilaire (FR); Greaves, Andrew, 77144 Montevrain (FR)
(74) Mandataire: Dossmann, Gérard

(56) Documents cités:
- EP-A- 1 609 456
- FR-A- 2 757 385
- FR-A- 2 822 696
- FR-A- 2 825 703

## Description

La présente demande a pour objet l'utilisation de composés azoïques polycationiques, à titre de colorants directs, dans des compositions tinctoriales pour la teinture des fibres kératiniques, et en particulier les cheveux humains. Elle vise également des compositions tinctoriales à base de ces colorants, ainsi que l'utilisation de ces compositions pour la teinture des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. Ces colorants, insolubles dans le milieu de teinture, sont piégés à l'intérieur du cheveu.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser poser pour permettre aux molécules colorées de pénétrer, par diffusion, à l'intérieur du cheveu, puis à rincer les fibres.

Contrairement aux compositions de teinture par oxydation, les compositions de teinture directes ou semi-permanentes peuvent avantageusement être mises en oeuvre sans la présence obligatoire d'un agent oxydant. Ces teintures peuvent être effectuées de manière répétée sans dégrader la fibre kératinique.

Il est connu par exemple d'utiliser des colorants directs nitrés benzéniques, anthraquinoniques, nitropyridiniques, azoïques, xanthéniques, acridiniques, aziniques ou triarylméthaniques.

Il en résulte des colorations souvent chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une faible puissance tinctoriale et une mauvaise tenue aux lavages ou à la transpiration. Ces colorants directs sont en outre généralement sensibles à la lumière car la résistance du chromophore vis-à-vis des attaques photochimiques est souvent faible, ce qui conduit à un affadissement de la coloration des cheveux dans le temps.

FR-A- 2 822 696 et FR-A-2825 703 décrivent des compositions pour la teinture des fibres kératiniques comprenant des colorants diazoïques dicationiques.

Par ailleurs, les colorants directs classiques ne sont pas toujours complètement inoffensifs, c'est pourquoi en cosmétique capillaire, on recherche des molécules colorantes de ce type toujours plus performantes en terme d'innocuité.

Il existe un réel besoin de disposer de compositions de teinture directe améliorées en terme de tenue aux shampooings et de montée du colorant.

De manière surprenante et avantageuse, la Demanderesse vient de découvrir que l'utilisation de composés azoïques polycationiques particuliers dans des compositions pour la teinture des fibres kératiniques, en particulier des fibres kératiniques humaines telles que les cheveux, permettait d'obtenir des compositions tinctoriales qui présentent ces améliorations.

Outre leur avantage en terme d'innocuité, les compositions selon la présente demande permettent l'obtention de teintures résistantes aux agents extérieurs (tels que le soleil, les intempéries), ainsi qu'aux shampooings et à la transpiration, et permettent d'obtenir des reflets intenses et tenaces sur les fibres.

En outre, ces compositions présentent une montée améliorée, une sélectivité réduite, ainsi qu'un bon profil toxicologique, la sélectivité étant la différence de montée entre les différentes parties d'un cheveu ou d'une chevelure.

La présente invention a ainsi pour objet l'utilisation à titre de colorant direct dans des compositions tinctoriales pour fibres kératiniques, en particulier les fibres kératiniques humaines telles que les cheveux, ou pour la fabrication de telles compositions, d'un composé de formule A-L-B particulier, dans laquelle A et B désignent un groupe à fonction colorante de type arylazoimidazolium, et L désigne un bras de liaison comprenant au moins un groupe cationique C.

L'invention a également pour objet une composition tinctoriale pour la teinture des fibres kératiniques, en particulier des fibres kératiniques humaines telles que les cheveux, comprenant dans un milieu approprié pour la teinture au moins un composé de formule A-L-B, tel que décrit ci-dessus, et au moins une base d'oxydation et/ou au moins un colorant direct additionnel.

Un autre objet de l'invention porte sur un procédé de teinture des fibres kératiniques, en particulier des fibres kératiniques humaines telles que les cheveux mettant en oeuvre les composés de formule A-L-B.

L'invention a également pour objet l'utilisation de la composition de la présente invention sur les fibres kératiniques, en particulier les fibres kératiniques humaines telles que les cheveux pour obtenir des teintures présentant une bonne résistance aux agents extérieurs et aux shampooings.

D'autres caractéristiques, aspects, objets et avantages de l'invention apparaîtront encore plus clairement à la lecture de la description et des exemples qui suivent.

A et B désignent, indépendamment, un groupe à fonction colorante porteur d'une charge cationique et de type arylazoimidazolium.

Le groupe L représente un « bras de liaison », qui relie les groupes A et B.

Le composé de formule A-L-B est choisi parmi les composés de formules (V) et (VI) suivantes : 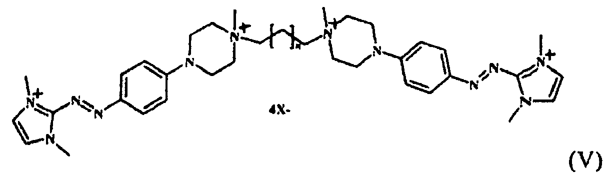 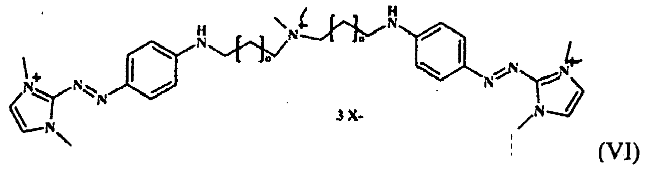 dans lesquelles :
X- désigne un anion d'origine minérale ou organique ; X⁻ peut être choisi notamment parmi les ions halogénures, et en particulier chlorure et iodure, les ions sulfate ou hydrogénosulfate, les ions méthosulfate, tosylate, carbonate ou hydrogénocarbonate, phosphate, nitrate, citrate, et
n est un entier allant de 0 à 20 et de préférence de 0 à 8,
à l'exclusion du dichlorure et bis(méthylsulfate) de 4-{4-[(E)-(1,3-diméthyl-1H-imidazol-3-ium-2-yl)diazényl]phényl}-1-[6-(4-{4-[(E)-(1,3-diméthyl-1H-imidazol-3-ium-2-yl)diazényl]phényl}-1-méthylpipérazine-1-ium-1-yl)hexyl]-1-méthylpipérazine-1-ium de formule (II) 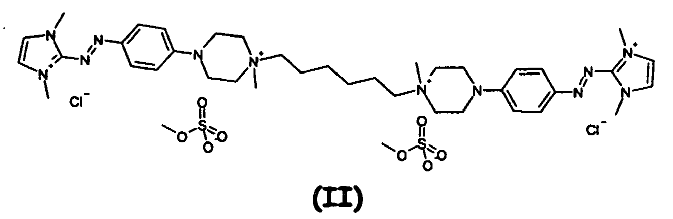
et du sel de dichlorure et de méthosulfate de 2-{(E)-[4-({3-[[3-({4-[(E)-(1,3-diméthyl-1H-imidazol-3-ium-2-yl)diazényl]phényl}amino)propyl](diméthyl)ammonio]propyl}amino)p hényl]diazényl}-1,3-diméthyl-1H-imidazol-3-ium de formule (IV).

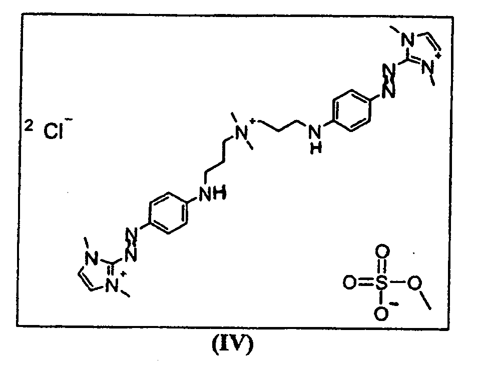

La composition selon la présente demande peut comprendre de 0,001 à 20%, de préférence de 0,01 à 10%, et de manière encore préférée 0,1 à 5% en poids de colorant(s) direct(s) de formule A-L-B par rapport au poids total de la composition.

La composition tinctoriale conforme à l'invention peut en outre contenir un ou plusieurs colorants directs additionnels autres que les colorants directs de formule A-L-B décrits ci-dessus, pouvant notamment être choisis parmi les colorants directs mentionnés plus haut, et notamment parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

On peut ainsi utiliser les colorants choisis parmi les colorants nitro aromatiques, anthraquinoniques, naphtoquinoniques, benzoquinoniques, naphtoquinoniques, azoïques, xanthéniques, triarylméthaniques, aziniques, thiaziniques, phénothiaziniques, diaziniques, phénodiaziniques, acridiniques, cyanineméthiniques, azométhiniques, nitrés, phtalocyaniques, indoaniliques, indophénoliques, et indoaminiques, et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-Bis(β -hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention, on peut citer les colorants azoïques cationiques décrits dans les demandes de brevets WO 95/15144, WO-95/01772 et EP-714954 dont le contenu fait partie intégrante de l'invention.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1 -naphtalène sulfonique.

Parmi les colorants directs quinoniques, on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels représentent de préférence de 0,001 à 20%, et encore plus préférentiellement de 0,01 à 10% en poids du poids total de la composition prête à l'emploi.

A titre d'exemple, les bases d'oxydation pouvant être présentes dans les composition selon l'invention sont choisies parmi les phénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4,5-diamino-1-(β-triéthoxyéthyl)pyrazole.

La ou les bases d'oxydation présentes dans la composition de l'invention sont en général présentes en quantité comprise allant de 0,001 à 20 % en poids environ du poids total de la composition tinctoriale, de préférence allant de 0,005 à 6 %.

La composition de la présente invention peut en outre comprendre un ou plusieurs précurseurs de colorant d'oxydation choisis parmi les coupleurs.

Les coupleurs peuvent être choisis parmi les coupleurs conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1 -N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en une quantité allant de 0,001 à 20 % en poids du poids total de la composition tinctoriale, de préférence allant de 0,01 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 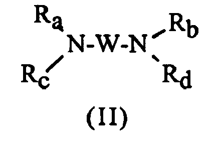 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La mise en oeuvre du procédé selon la présente demande comprend l'application d'une composition tinctoriale selon la présente demande sur les fibres kératiniques, puis une étape consistant à laisser poser pendant une période suffisante pour permettre la coloration des cheveux, cette période est généralement comprise entre 5 minutes et 1 heure et de préférence entre 15 minutes et 1 heure.

Le procédé de teinture des fibres kératiniques peut comprendre une étape mettant en oeuvre un agent oxydant à pH acide, neutre ou alcalin. L'agent oxydant peut être ajouté à la composition de l'invention juste au moment de l'emploi ou il peut être mis en oeuvre à partir d'une composition oxydante le contenant, appliqué simultanément ou séquentiellement à la composition de l'invention.

Dans une variante de ce procédé, la composition comprenant le ou les composés de formule A-L-B contient au moins un précurseur de colorant d'oxydation.

Selon un mode de réalisation particulier, la composition selon la présente invention comprenant le composé de formule A-L-B et éventuellement au moins un précurseur de colorant d'oxydation est mélangée, de préférence au moment de l'emploi, à une composition contenant, dans un milieu approprié pour la teinture, au moins un agent oxydant. Le mélange obtenu est ensuite appliqué sur les fibres kératiniques. Après un temps de pause de 5 minutes à 1 heure environ, de préférence 15 minutes à 1 heure environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et de préférence entre 5 et 11 environ et de manière encore plus préférée entre 6 et 8,5. Il peut être ajusté à la valeur désirée au moyen d'agents régulateurs de pH, acidifiants ou alcalinisants habituellement utilisés en teinture des fibres kératiniques et tels que définis précédemment.

La composition appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

La présente demande a également pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour obtenir la coloration désirée.

## Revendications

1. Utilisation à titre de colorant direct dans des compositions tinctoriales pour fibres kératiniques, en particulier les fibres kératiniques humaines telles que les cheveux, ou pour la fabrication de telles compositions, d'un composé de formule A-L-B, dans laquelle A et B désignent un groupe à fonction colorante de type arylazoimidazolium, et L désigne un bras de liaison comprenant au moins un groupe cationique C, le composé de formule A-L-B étant choisi parmi les composés de formules (V) et (VI) suivantes : 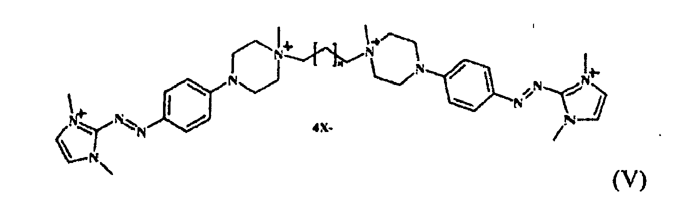 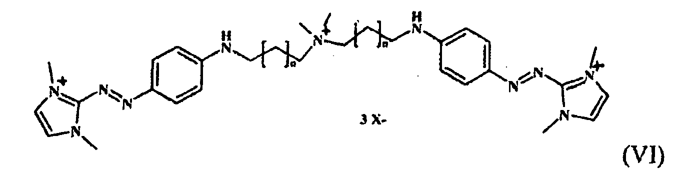 dans lesquelles :
X⁻ désigne un anion d'origine minérale ou organique ; et
n est un entier allant de 0 à 20 et de préférence de 0 à 8 ;
à l'exclusion du dichlorure et bis(méthylsulfate) de 4-{4-[(E)-(1,3-diméthyl-1H-imidazol-3-ium-2-yl)diazényl]phényl}-1-[6-(4-{4-[(E)-(1,3-diméthyl-1H-imidazol-3-ium-2-yl)diazényl]phényl}-1-méthylpipérazine-1-ium-1-yl)hexyl]-1-méthylpipérazine-1-ium, et du dichlorure et méthosulfate de 2-{(E)-[4-({3-[[3-({4-[(E)-(1,3-diméthyl-1H-imidazol-3-ium-2-yl)diazényl]phényl}amino)propyl](diméthyl)ammonio]propyl}amino)p hényl]diazényl}-1,3-diméthyl-1H-imidazol-3-ium.

2. Utilisation selon la revendication 1, **caractérisée en ce que** X⁻ est choisi parmi les ions halogénures, les ions sulfate ou hydrogénosulfate, les ions méthosulfate, tosylate, carbonate ou hydrogénocarbonate, phosphate, nitrate, citrate.

3. Composition tinctoriale pour la teinture des fibres kératiniques, en particulier des fibres kératiniques humaines telles que les cheveux comprenant dans un milieu approprié pour la teinture au moins un composé de formule A-L-B, dans laquelle A et B désignent un groupe à fonction colorante de type arylazoimidazolium, et L désigne un bras de liaison comprenant au moins un groupe cationique C, la composition comprenant en outre au moins une base d'oxydation et/ou au moins un colorant direct additionnel ; le composé de formule A-L-B étant choisi parmi les composés de formules (V) et (VI) suivantes : 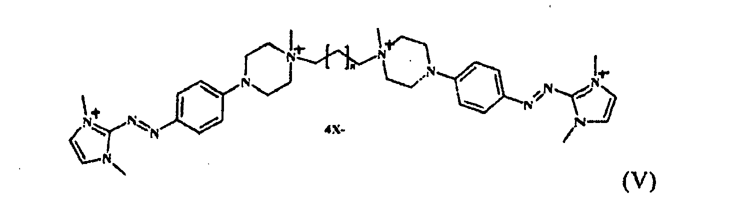 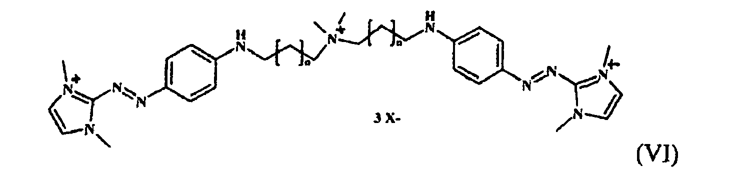 dans lesquelles :
X⁻ désigne un anion d'origine minérale ou organique ; et
n est un entier allant de 0 à 20 et de préférence de 0 à 8 ;
à l'exclusion du dichlorure et bis(méthylsulfate) de 4-{4-[(E)-(1,3-diméthyl-1H-imidazol-3-ium-2-yl)diazényl]phényl}-1-[6-(4-{4-[(E)-(1,3-diméthyl-1H-imidazol-3-ium-2-yl)diazényl]phényl}-1-méthylpipérazine-1-ium-1-yl)hexyl]-1-méthylpipérazine-1-ium, et du dichlorure et méthosulfate de 2-{(E)-[4-({3-[[3-({4-[(E)-(1,3-diméthyl-1H-imidazol-3-ium-2-yl)diazényl]phényl}amino)propyl](diméthyl)ammonio]propyl}amino)p hényl]diazényl}-1,3-diméthyl-1H-imidazol-3-ium.

4. Composition selon la revendication 3, **caractérisée en ce que** X⁻ est choisi parmi les ions halogénures, les ions sulfate ou hydrogénosulfate, les ions méthosulfate, tosylate, carbonate ou hydrogénocarbonate, phosphate, nitrate, citrate.

5. Composition selon la revendication 3 ou 4, **caractérisée en ce qu'**elle comprend de 0,001 à 20%, de préférence de 0,01 à 10%, et de manière encore préférée de 0,1 à 5% en poids de colorant(s) direct(s) de formule A-L-B par rapport au poids total de la composition.

6. Composition tinctoriale selon l'une des revendications 3 à 5, **caractérisée en ce que** la base d'oxydation est choisie parmi les paraphénylènediamines, les bis-phénylalkylènediamines, les paraaminophénols, les orthoaminophénols, les bases hétérocycliques et leurs sels d'addition.

7. Composition tinctoriale selon l'une des revendications 3 à 6, **caractérisée en ce que** la ou les bases d'oxydation sont présentes en une quantité comprise entre 0,001 à 20 % en poids et de préférence entre 0,005 et 6 % en poids par rapport au poids total de la composition.

8. Composition selon l'une des revendications 3 à 7, **caractérisée en ce que** le colorant direct additionnel est choisi parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

9. Composition selon la revendication 8, **caractérisée en ce que** le colorant direct additionnel représente de 0,001 à 20%, et de préférence de 0,01 à 10% en poids du poids total de la composition.

10. Composition selon l'une des revendications 3 à 9, **caractérisée en ce qu'**elle contient au moins un précurseur de colorant d'oxydation choisi parmi les coupleurs.

11. Composition selon la revendication 10, **caractérisée en ce que** le coupleur est choisi parmi les métaphénylènediamines, les métaaminophénols, les métadiphénols, les coupleurs naphtaléniques, les coupleurs hétérocycliques et leurs sels d'addition.

12. Composition selon la revendication 10, **caractérisée en ce que** le coupleur est choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol le 6-hydroxy indole, le 4-hydroxy indole, le 4-hydroxy N-méthyl iodole, la 2-amino-3-hydroxy pyridine, la 6- hydroxy benzomorpholine la 3,5-diamino-2,6-diméthoxypyridine, le 1-N-(13-hydroxyéthyl)amino-3,4-méthylène dioxybenzène, le 2,6-bis-(β-hydroxyéthylamino)toluène et leurs sels d'addition.

13. Composition selon l'une des revendications 10 à 12, **caractérisée en ce que** le ou les coupleurs sont présents en une quantité comprise entre 0,001 et 20 % de préférence entre 0,005 et 6 % en poids par rapport au poids total de la composition.

14. Composition selon l'une des revendications 3 à 13, **caractérisée en ce qu'**elle comprend au moins un adjuvant choisi parmi les tensioactifs anioniques, cationiques, non ioniques, amphotères, zwitterioniques ou leurs mélanges, les polymères anioniques, cationiques, non ioniques, amphotères, zwitterioniques ou leurs mélanges, les agents épaississants minéraux ou organiques, les agents antioxydants, les agents de pénétration, les agents séquestrants, les parfums, les tampons, les agents dispersants, les agents conditionneurs, les agents filmogènes, les céramides, les agents conservateurs, les agents opacifiants.

15. Composition selon l'une des revendications 3 à 14, **caractérisée en ce qu'**elle comprend au moins un agent oxydant choisi parmi le peroxyde d'hydrogène, le peroxyde d'urée, les bromates de métaux alcalins, les persels, les peracides et les enzymes oxydases.

16. Composition selon la revendication 15, **caractérisée en ce que** l'agent oxydant est le peroxyde d'hydrogène.

17. Procédé de teinture des fibres kératiniques, **caractérisé en ce qu'**il comprend l'application d'une composition selon l'une des revendications 3 à 16, sur les fibres kératiniques, puis une étape consistant à laisser poser pendant une période comprise entre 5 minutes et une heure, de préférence entre 15 minutes et 1 heure.

18. Utilisation d'une composition selon l'une des revendications 3 à 16 pour la teintures des fibres kératiniques, en particulier des fibres kératiniques humaines telles que les cheveux.

19. Utilisation d'une composition selon l'une , des revendications 3 à 16 sur les fibres kératiniques, en particulier les fibres kératiniques humaines telles que les cheveux, pour obtenir des teintures présentant une bonne résistance aux agents extérieurs et aux shampoings.

## Claims

1. Use, as a direct dye in dye compositions for keratin fibres, in particular human keratin fibres such as the hair, or for the manufacture of such compositions, of a compound of formula A-L-B, in which A and B denote a group containing a colouring function of arylazoimidazolium type and L denotes a linker comprising at least one cationic group C, the compound of formula A-L-B being chosen from the compounds having the following formulae (V) and (VI): 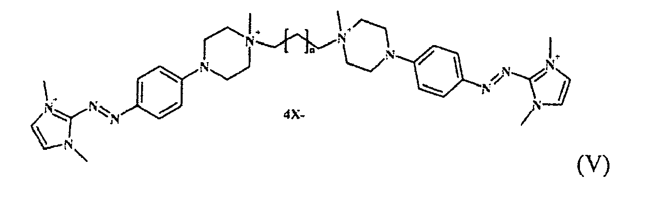 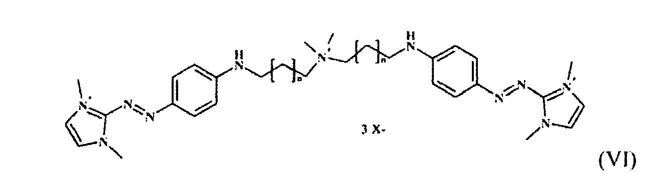 in which:
x⁻ denotes an anion of mineral or organic origin; and
n is an integer ranging from 0 to 20 and preferably from 0 to 8;
to the exclusion of 4-{4-[(E)-(1,3-dimethyl-1H-imidazol-3-ium-2-yl)diazenyl]phenyl}-1-[6-(4-{4-[(E)-(1,3-dimethyl-1H-imidazol-3-ium-2-yl)diazenyl]phenyl}-1-methylpiperazin-1-ium-1-yl)hexyl]-1-methylpiperazin-1-ium bis-(methyl sulfate) dichloride, and of 2-{(E)-[4-({3-[[3-({4-[(E)-(1,3-dimethyl-1H-imidazol-3-ium-2-yl)diazenyl]phenyl}amino)propyl](dimethyl) ammonio]propyl}amino)phenyl]diazenyl}-1,3-dimethyl-1H-imidazol-3-ium methosulfate dichloride.

2. Use according to Claim 1, **characterized in that** X⁻ is chosen from halide ions, sulfate or hydrogen sulfate ions, and methosulfate, tosylate, carbonate or hydrogen carbonate, phosphate, nitrate and citrate ions.

3. Dye composition for dyeing keratin fibres, in particular human keratin fibres such as the hair, comprising, in a medium that is suitable for dyeing, at least one compound of formula A-L-B, in which A and B denote a group containing a colouring function of arylazoimidazolium type, and L denotes a linker comprising at least one cationic group C,
the composition further comprising at least one oxidation base and/or at least one additional direct dye; the compound of formula A-L-B being chosen from the compounds having the following formulae (V) and (VI): 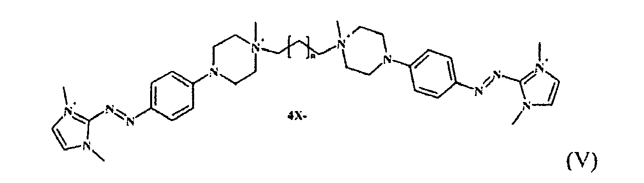 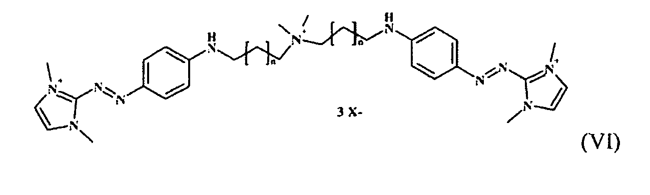 in which:
x⁻ denotes an anion of mineral or organic origin, and
n is an integer ranging from 0 to 20 and preferably from 0 to 8;
to the exclusion of 4-{4-[(E)-(1,3-dimethyl-1H-imidazol-3-ium-2-yl)diazenyl]phenyl}-1-[6-(4-{4-[(E)-(1,3-dimethyl-1H-imidazol-3-ium-2-yl)diazenyl]phenyl}-1-methylpiperazin-1-ium-1-yl)hexyl]-1-methylpiperazin-1-ium bis(methyl sulfate) dichloride, and of 2-{(E)-[4-({3-[[3-({4-[(E)-(1,3-dimethyl-1H-imidazol-3-ium-2-yl)diazenyl]phenyl}amino)propyl](dimethyl) ammonio]propyl}amino)phenyl]diazenyl}-1,3-dimethyl-1H-imidazol-3-ium methosulfate dichloride.

4. Composition according to Claim 3, **characterized in that** X⁻ is chosen from halide ions, sulfate or hydrogen sulfate ions, and methosulfate, tosylate, carbonate or hydrogen carbonate, phosphate, nitrate and citrate ions.

5. Composition according to Claim 3 or 4, **characterized in that** it comprises from 0.001% to 20%, preferably from 0.01% to 10% and even more preferably from 0.1% to 5% by weight of direct dye(s) of formula A-L-B relative to the total weight of the composition.

6. Dye composition according to one of Claims 3 to 5, **characterized in that** the oxidation base is chosen from para-phenylenediamines, bis(phenyl)alkylenediamines, para-aminophenols, ortho-aminophenols, heterocyclic bases, and the addition salts thereof.

7. Dye composition according to one of Claims 3 to 6, **characterized in that** the oxidation base(s) is (are) present in an amount of between 0.001% and 20% by weight and preferably between 0.005% and 6% by weight relative to the total weight of the composition.

8. Composition according to one of Claims 3 to 7, **characterized in that** the additional direct dye is chosen from neutral, acidic or cationic nitrobenzene-based dyes, neutral, acidic or cationic azo direct dyes, neutral, acidic or cationic quinonic and in particular anthraquinonic direct dyes, azine-based direct dyes, triarylmethane-based direct dyes, indoamine-based direct dyes and natural direct dyes.

9. Composition according to Claim 8, **characterized in that** the additional direct dye represents from 0.001% to 20% and preferably from 0.01% to 10% by weight relative to the total weight of the composition.

10. Composition according to one of Claims 3 to 9, **characterized in that** it contains at least one oxidation dye precursor chosen from couplers.

11. Composition according to Claim 10, **characterized in that** the coupler is chosen from meta-phenylenediamines, meta-aminophenols, meta-diphenols, naphthalene-based couplers and heterocyclic couplers, and the addition salts thereof.

12. Composition according to Claim 10, **characterized in that** the coupler is chosen from 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureido-aniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxy-benzomorpholine, 3,5-diamino-2,6-dimethoxy-pyridine, 1-N-(β-hydroxyethyl)amino-3,4-methylenedioxybenzene, 2,6-bis(β-hydroxyethylamino)toluene, and the addition salts thereof.

13. Composition according to one of Claims 10 to 12, **characterized in that** the coupler(s) is (are) present in an amount of between 0.001% and 20% and preferably between 0.005% and 6% by weight relative to the total weight of the composition.

14. Composition according to one of Claims 3 to 13, **characterized in that** it comprises at least one adjuvant chosen from anionic, cationic, nonionic, amphoteric or zwitterionic surfactants or mixtures thereof, anionic, cationic, nonionic, amphoteric or zwitterionic polymers or mixtures thereof, mineral or organic thickeners, antioxidants, penetrating agents, sequestering agents, fragrances, buffers, dispersants, conditioners, film-forming agents, ceramides, preserving agents, opacifiers.

15. Composition according to one of Claims 3 to 14, **characterized in that** it comprises at least one oxidizing agent chosen from hydrogen peroxide, urea peroxide, alkali metal bromates, persalts, peracids and oxidase enzymes.

16. Composition according to Claim 15, **characterized in that** the oxidizing agent is hydrogen peroxide.

17. Process for dyeing keratin fibres, **characterized in that** it comprises the application of the composition according to one of Claims 3 to 16 to the keratin fibres, followed by a step consisting in leaving it for a period of between 5 minutes and one hour, preferably between 15 minutes and one hour.

18. Use of a composition according to one of Claims 3 to 16 for dyeing keratin fibres, in particular human keratin fibres such as the hair.

19. Use of a composition according to one of Claims 3 to 16 on keratin fibres, in particular human keratin fibres such as the hair, to obtain dyeing results that show good resistance to external agents and to shampoos.

## Patentansprüche

1. Verwendung einer Verbindung der Formel A-L-B, wobei A und B eine Gruppe mit Farbstofffunktion vom Arylazoimidazoliumtyp bedeuten und L eine Verbindungsgruppe ist, die mindestens eine kationische Gruppe C enthält, als Direktfarbstoff in Zusammensetzungen zum Färben von Keratinfasern und insbesondere menschlichen Keratinfasern, wie Haaren, oder für die Herstellung solcher Zusammensetzungen, wobei die Verbindung der Formel A-L-B unter den Verbindungen der folgenden Formeln (V) und (VI) ausgewählt sind: 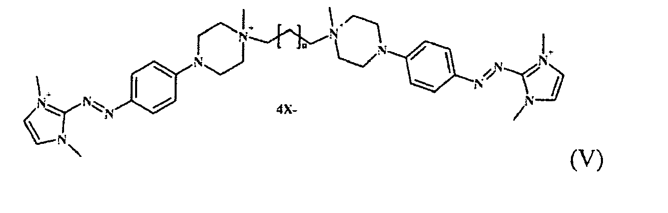 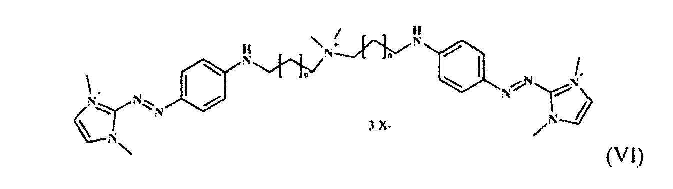 worin bedeuten:
X⁻ ein Anion anorganischer oder organischer Herkunft; und
n eine ganze Zahl von 0 bis 20 und vorzugsweise 0 bis 8;
wobei das 4-{4-[(E)-(1,3-Dimethyl-1H-imidazol-3-ium-2-yl)diazenyl]phenyl}-1-[6-(4-{4-[(E)-(1,3-dimethyl-1H-imidazol-3ium-2-yl)diazenyl]phenyl}-1-methylpiperazin-1-ium-1-yl)hexyl]-1-methylpiperazin-1-ium-dichlorid-bis(methylsulfat) und das 2-{(E)-[4-({3-[[3-[{4-[(E)-(1,3-dimethyl-1H-imidazol-3-ium-2-yl)diazenyl]phenyl}amino)propyl](dimethyl)ammonio]propyl} amino)phenyl]diazenyl}-1,3-dimethyl-1H-imidazol-3-ium-dichlorid-methosulfat ausgenommen sind.

2. Verwendung nach Anspruch 1, **dadurch gekennzeichnet, dass** X⁻ unter den Halogeniden, Sulfat oder Hydrogensulfat, Methosulfat, Tosylat, Carbonat oder Hydrogencarbonat, Phosphat, Nitrat und Citrat ausgewählt ist.

3. Farbmittelzusammensetzung zum Färben von Keratinfasern und insbesondere menschlichen Keratinfasern, wie Haaren, die in einem zum Färben geeigneten Medium mindestens eine Verbindung der Formel A-L-B enthält, wobei A und B eine Gruppe mit Farbstofffunktion vom Arylazoimidazoliumtyp bedeuten und L eine Verbindungsgruppe ist, die mindestens eine kationische Gruppe C aufweist, wobei die Zusammensetzung ferner mindestens eine Oxidationsbase und/oder mindestens einen ergänzenden Direktfarbstoff enthält und wobei die Verbindung der Formel A-L-B unter den Verbindungen der folgenden Formeln (V) und (VI) ausgewählt ist: 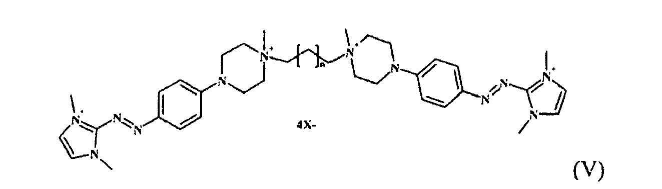 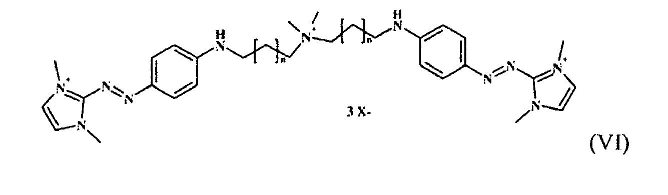 worin bedeuten:
X⁻ ein Anion anorganischer oder organischer Herkunft; und
n eine ganze Zahl von 0 bis 20 und vorzugsweise 0 bis 8;
wobei das 4-{4-[(E)-(1,3-Dimethyl-1H-imidazol-3-ium-2-yl)diazenyl]phenyl}-1-[6-(4-{4-[(E)-(1,3-dimethyl-1 H-imidazol-3-ium-2-yl) diazenyl]phenyl}-1-methylpiperazin-1-ium-1-yl)hexyl]-1-methylpiperazin-1-ium-dichlorid-bis(methylsulfat) und das 2-{(E)-[4-({3-[[3-({4-[(E)-(1,3-dimethyl-1H-imidazol-3-ium-2-yl)diazenyl]phenyl}amino)propyl](dimethyl)-ammonio]propyl}amino)phenyl]diazenyl}-1,3-dimethyl-1H-imidazol-3-ium-dichlorid-methosulfat ausgenommen sind.

4. Zusammensetzung nach Anspruch 3, **dadurch gekennzeichnet, dass** X⁻ unter den Halogeniden, Sulfat oder Hydrogensulfat, Methosulfat, Tosylat, Carbonat oder Hydrogencarbonat, Phosphat, Nitrat und Citrat ausgewählt ist.

5. Zusammensetzung nach Anspruch 3 oder 4, **dadurch gekennzeichnet, dass** sie 0,001 bis 20 %, vorzugsweise 0,01 bis 10 % und noch bevorzugter 0,1 bis 5 Gew.-% Direktfarbstoff(e) der Formel A-L-B, bezogen auf das Gesamtgewicht der Zusammensetzung, enthält.

6. Farbmittelzusammensetzung nach einem der Ansprüche 3 bis 5, **dadurch gekennzeichnet, dass** die Oxidationsbase unter den *para*-Phenylendiaminen, Bisphenylalkylendiaminen, *para-*Aminophenolen, *ortho*-Aminophenolen, heterocyclischen Basen, und deren Additionssalzen ausgewählt ist.

7. Farbmittelzusammensetzung nach einem der Ansprüche 3 bis 6, **dadurch gekennzeichnet, dass** die Oxidationsbase oder die Oxidationsbasen in einer Menge von 0,001 bis 20 Gew.-% und vorzugsweise 0,005 bis 6 Gew.-%, bezogen auf das Gesamtgewicht der Zusammensetzung, enthalten sind.

8. Zusammensetzung nach einem der Ansprüche 3 bis 7, **dadurch gekennzeichnet, dass** der ergänzende Direktfarbstoff unter den neutralen, sauren oder kationischen nitrierten Farbstoffen aus der Benzolreihe, neutralen, sauren oder kationischen direktziehenden Azofarbstoffen, neutralen, sauren oder kationischen direktziehenden Chinon-Farbstoffen und insbesondere Anthrachinon-Farbstoffen, direktziehenden Azinfarbstoffen, direktziehenden Triarylmethan-Farbstoffen, direktziehenden Indoamin-Farbstoffen und direktziehenden natürlichen Farbstoffen ausgewählt ist.

9. Zusammensetzung nach Anspruch 8, **dadurch gekennzeichnet, dass** der ergänzende Direktfarbstoff 0,001 bis 20 % und vorzugsweise 0,01 bis 10 Gew.-% des Gesamtgewichts der Zusammensetzung ausmacht.

10. Zusammensetzung nach einem der Ansprüche 3 bis 9, **dadurch gekennzeichnet, dass** sie mindestens ein Farbstoffvorprodukt eines Oxidationsfarbstoffes enthält, das unter den Kupplern ausgewählt ist.

11. Zusammensetzung nach Anspruch 10, **dadurch gekennzeichnet, dass** der Kuppler unter den *meta*-Phenylendiaminen, *meta-*Aminophenolen, *meta*-Diphenolen, Naphthalinkupplern, heterocyclischen Kupplern und deren Additionssalzen ausgewählt ist.

12. Zusammensetzung nach Anspruch 10, **dadurch gekennzeichnet, dass** der Kuppler unter 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis(2,4-diamino-phenoxy)propan, 3-Ureidoanilin, 3-Ureido-1-dimethylaminobenzol, Sesamol, 1-β-Hydroxyethylamino-3,4-methylendioxybenzol, α-Naphthol, 2-Methyl-1-naphthol, 6-Hydroxyindol, 4-Hydroxyindol, 4-Hydroxy-N-methylindol, 2-Amino-3-hydroxy-pyridin, 6-Hydroxybenzomorpholin, 3,5-Diamino-2,6-dimethoxy-pyridin, 1-N-(β-Hydroxyethyl)amino-3,4-methylendioxybenzol, 2,6-Bis(β-hydroxyethylamino)toluol, und ihren Additionssalzen ausgewählt ist.

13. Zusammensetzung nach einem der Ansprüche 10 bis 12, **dadurch gekennzeichnet, dass** der Kuppler oder die Kuppler in einer Menge von 0,001 bis 20 %, vorzugsweise 0,005 bis 6 Gew.-%, bezogen auf das Gesamtgewicht der Zusammensetzung, enthalten sind.

14. Zusammensetzung nach einem der Ansprüche 3 bis 13, **dadurch gekennzeichnet, dass** sie mindestens einen Zusatzstoff enthält, der unter den anionischen, kationischen, nichtionischen, amphoteren, zwitterionischen grenzflächenaktiven Stoffen oder deren Gemischen, anionischen, kationischen, nichtionischen, amphoteren, zwitterionischen Polymeren oder deren Gemischen, anorganischen oder organischen Verdickungsmitteln, Antioxidantien, Penetrationsmitteln, Maskierungsmitteln, Parfums, Puffern, Dispergiermitteln, Konditioniermitteln, Filmbildnern, Ceramiden, Konservierungsmitteln und Trübungsmitteln ausgewählt ist.

15. Zusammensetzung nach einem der Ansprüche 3 bis 14, **dadurch gekennzeichnet, dass** sie mindestens ein Oxidationsmittel enthält, das unter Wasserstoffperoxid, Harnstoffperoxid, Alkalimetallbromaten, Persalzen, Persäuren und Oxidasen ausgewählt ist.

16. Zusammensetzung nach Anspruch 15, **dadurch gekennzeichnet, dass** es sich bei dem Oxidationsmittel um Wasserstoffperoxid handelt.

17. Verfahren zum Färben von Keratinfasern, **dadurch gekennzeichnet, dass** es das Aufbringen einer Zusammensetzung nach einem der Ansprüche 3 bis 16 auf die Keratinfasern und anschließend einen Schritt umfasst, der darin besteht, sie während einer Zeitspanne von 5 Minuten bis 1 Stunde und vorzugsweise 15 Minuten bis 1 Stunde einwirken zu lassen.

18. Verwendung einer Zusammensetzung nach einem der Ansprüche 3 bis 16 zum Färben von Keratinfasern und insbesondere menschlichen Keratinfasern wie Haaren.

19. Verwendung einer Zusammensetzung nach einem der Ansprüche 3 bis 16 an Keratinfasern und insbesondere menschlichen Keratinfasern, wie Haaren, um Färbungen zu erhalten, die gegenüber von außen einwirkenden Agentien und Haarwäschen eine hohe Beständigkeit aufweisen.
